# FASCICULE DE BREVET EUROPEEN

(11) **EP 1 779 843 B1**
(45) Date de publication et mention de la délivrance du brevet: **27.01.2010**
(21) Numéro de dépôt: 06291671.3
(22) Date de dépôt: 26.10.2006
(51) Int. Cl.: A61K 8/49, A61K 8/46, A61K 8/44, A61K 8/73, A61K 8/81, A61Q 5/02

(54) **Composition de lavage des matières kératiniques et procédé de traitement cosmétique mettant en oeuvre ladite composition**
Waschzusammensetzung für Keratinfasern und Verfahren zur kosmetischen Behandlung
Washing composition for keratinous materials and process for cosmetic treatment therefore

(30) Priorité: 28.10.2005 FR 0511096
(43) Date de publication de la demande: 02.05.2007
(73) Titulaire: L'Oréal, 75008 Paris (FR)
(72) Inventeur: Decoster, Sandrine, 95210 Saint-Gratien (FR); Thomas, Béatrice, 92130 Issy les Moulineaux (FR)
(74) Mandataire: Zapalowicz, Francis

(56) Documents cités:
- EP-A- 1 043 010
- WO-A-01/39735
- WO-A-97/26860
- FR-A1- 2 387 031
- US-A1- 2003 022 799
- PATEL C U: "ANTI-STATIC PROPERTIES OF SOME CATIONIC POLYMERS USED IN HAIR CARE PRODUCTS PROPRIETES ANTI-STATIQUES DE CERTAINS POLYMERES CATIONIQUES UTILISES DANS LES PRODUITS DE SOINS POUR LES CHEVEUX" INTERNATIONAL JOURNAL OF COSMETIC SCIENCE, BLACKWELL SCIENTIFIC PUBLICATIONS, OXFORD, GB, vol. 5, no. 5, 1983, pages 181-188, XP008021180 ISSN: 0142-5463

## Description

La présente invention est relative à une composition cosmétique améliorée pour le lavage des matières kératiniques, en particulier des cheveux et/ou de la peau, et qui comprend une combinaison originale d'agents tensioactifs et de polymère(s). Plus précisément, la présente invention concerne une composition cosmétique pour le lavage des matières kératiniques, comprenant au moins deux tensioactifs anioniques différents, au moins un tensioactif amphotère ou zwittérionique, au moins un ester de sorbitan oxyéthyléné, et au moins un polymère cationique et/ou au moins un polymère amphotère ou zwittérionique. La présente invention concerne également un procédé de traitement cosmétique mettant en oeuvre une telle composition cosmétique.

De nombreuses compositions douces de lavage ont été décrites dans l'art antérieur.

Ainsi, la demande de brevet FR 2 804 020 décrit des compositions de lavage, notamment des shampooings, comprenant au moins un tensioactif détergent et au moins un ester d'acide gras et de sorbitan oxyéthyléné ayant un nombre de moles d'oxyéthylène inférieur ou égal à 10.

Le brevet US 5 270 035 décrit des compositions de traitement des cheveux comprenant, en milieu aqueux, du cocoamphodiacétate de sodium, associé à un agent émulsifiant, un tensioactif et une substance astringente à base d'extraits de plantes.

La demande de brevet EP 155 737 décrit des compositions de shampooings faiblement irritantes, destinées aux personnes présentant des dermatoses. Ces compositions comprennent :
- une bétaïne,
- au moins deux tensioactifs anioniques dont du laurylsulfate de triéthanolamine et un ou plusieurs composés choisis parmi les alkylsulfates de métaux alcalins, les N-acylamino acides ou leurs sels et les alkyl éther sulfonates polyoxyéthylène,
- au moins un tensioactif non ionique choisi parmi les alcanolamides d'acides gras et les produits polyoxyéthylénés solubles dans l'eau.

La demande de brevet EP 453 238 décrit des compositions de shampooings douces dont le pouvoir moussant est amélioré. Ces compositions comprennent, en milieu aqueux, de 8 à 25 % en poids d'un mélange de tensioactifs constitué de :
- un tensioactif anionique,
- un tensioactif amphotère, à l'exception des bétaïnes contenant du phosphore,
- un tensioactif non ionique oxyalkylé ou glycosidique ayant une HLB d'au moins 8.

La demande de brevet WO 02/05758 décrit une composition de nettoyage automoussante, dont le pouvoir moussant et la facilité d'application sont améliorés. Cette composition comprend au moins un agent automoussant, associé à un mélange de tensioactifs comprenant au moins un tensioactif anionique, au moins un tensioactif amphotère, et, en option, au moins un tensioactif non ionique. Cette composition peut en outre comprendre un ou plusieurs agents conditionneurs cationiques tels que par exemple des dérivés cationiques de cellulose, des dérivés cationiques de guar, des dérivés et copolymères de chlorure de diallyldiméthylammonium.

Le brevet CA 1 077 849 décrit des compositions détergentes et des shampooings diminuant les risques d'irritation oculaire et dont le pouvoir moussant (volume et stabilité de la mousse) est amélioré. Ces compositions comprennent une bétaïne tensioactive, un tensioactif anionique, et un tensioactif non-ionique constitué d'un dérivé polyoxyéthyléné, soluble dans l'eau, d'une base hydrophobe, avec un rapport molaire entre la quantité de bétaïne et la quantité de tensioactif anionique comprise entre 0,9 / 1 et 1,1 / 1.

Enfin, la demande de brevet WO 03/057185 décrit des compositions cosmétiques ou dermatologiques pour le soin ou le nettoyage de la peau et des cheveux, qui permettent de combiner douceur et bon pouvoir nettoyant. Ces compositions sont basées sur une combinaison synergique de tensioactifs anioniques et non ioniques comprenant des alkylpolyglycosides, des alkykl(éther)sulfates, des esters de sorbitan polyéthylèneglycol et des alkyl citrate sulfosuccinates.

Toutefois, les compositions décrites dans l'art antérieur présentent certaines insuffisances. En particulier, les shampooings les plus performants peuvent provoquer des picotements dans l'oeil lorsque le produit dilué coule dans la sphère oculaire, ce qui arrive fréquemment chez les enfants. Par ailleurs, bon nombre de ces shampooings provoquent, chez les personnes présentant une peau sensible, des réactions d'inconfort telles que des rougeurs, des démangeaisons, des picotements.

Les compositions douces proposées dans l'art antérieur présentent à l'inverse des qualités d'usage non satisfaisantes en terme de viscosité et de qualité de mousse, et des propriétés cosmétiques insuffisantes, notamment en termes de douceur et, en ce qui concerne les cheveux, de démêlage, de lissage, de coiffant et de volume.
La Demanderesse a maintenant découvert de manière surprenante qu'une combinaison particulière de certains agents tensioactifs, associés à au moins un polymère cationique et/ou amphotère ou zwittérionique, permet de formuler des compositions cosmétiques particulièrement douces, présentant néanmoins d'excellentes propriétés cosmétiques. En particulier, la présence d'un tensioactif anionique sous forme de sel de magnésium dans de telles compositions permet d'obtenir des compositions présentant des propriétés cosmétiques améliorées par rapport à des compositions de l'art antérieur ne comprenant pas de tels sels.

On a constaté en effet que les compositions selon la présente invention apportent un meilleur lissage visuel et que les cheveux traités sont plus souples et ont un meilleur toucher. En particulier, elles permettent d'améliorer le démêlage, d'augmenter la légèreté et le lissage des cheveux humides ; et sur cheveux séchés, elles apportent plus de brillance, augmentent la souplesse et le lissage des cheveux visuellement et au toucher.

Ainsi, les compositions selon l'invention permettent de diminuer les réactions d'inconfort au niveau de la peau et du cuir chevelu, et possèdent un excellent niveau de tolérance oculaire. Parallèlement, elles présentent de bonnes qualités d'usage, et de meilleures propriétés cosmétiques, notamment en termes de douceur et, en ce qui concerne les cheveux outre la douceur, de démêlage, de lissage, de brillance, de souplesse, de coiffant et de volume.

La présente invention a donc pour objet une composition cosmétique pour le lavage des matières kératiniques comprenant, dans un milieu aqueux :
(a) au moins un premier tensioactif anionique sous forme de sel de magnésium,
(b) au moins un deuxième tensioactif anionique différent du premier et qui n'est pas sous forme de sel de magnésium,
(c) au moins un tensioactif amphotère ou zwittérionique,
(d) au moins un ester de sorbitan oxyéthyléné, et
(e) au moins un polymère cationique et/ou au moins un polymère amphotère ou zwittérionique.

Un autre objet de l'invention est un procédé de traitement cosmétique mettant en oeuvre ladite composition.

D'autres objets et caractéristiques, aspects et avantages de l'invention apparaîtront plus clairement à la lecture de la description et des exemples qui suivent.

Selon l'invention, la composition cosmétique comprend au moins un premier tensioactif anionique sous forme de sel de magnésium. Ledit premier tensioactif anionique est avantageusement choisi parmi les sels de magnésium des composés suivants: les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, et leurs mélanges, les groupes alkyle et acyle de tous ces composés comportant de 6 à 24 atomes de carbone et le groupe aryle désignant de préférence un groupe phényle ou benzyle.

De préférence, ledit premier tensioactif anionique comprend au moins un alkyléthersulfate de magnésium contenant de 1 à 16 motifs d'oxyde d'éthylène. Le groupe alkyle est avantageusement en C₈₋₃₀, et de préférence en C₁₂₋₂₄.

Citons par exemple les lauryl éther sulfates de magnésium contenant de 1 à 16 motifs d'oxyde d'éthylène, et plus particulièrement de 1 à 5 motifs d'oxyde d'éthylène.

La composition selon l'invention comprend préférentiellement au moins 0,1 % en poids dudit premier tensioactif anionique par rapport au poids total de la composition. De préférence, elle comprend de 0,1 à 10 % en poids dudit premier tensioactif anionique, plus préférentiellement de 0,2 à 8% en poids, et encore plus préférentiellement de 0,3 à 2 % en poids, par rapport au poids total de la composition.

La composition objet de la présente invention comprend en outre au moins un deuxième tensioactif anionique, différent dudit premier tensioactif anionique. Ledit deuxième tensioactif anionique n'est pas sous forme de sel de magnésium.

Les tensioactifs anioniques pouvant être utilisés sont notamment choisis parmi les sels, en particulier les sels de métaux alcalins ou alcalino-terreux autres que le magnésium, tels que les sels de sodium, les sels d'ammonium, les sels d'amines, les sels d'aminoalcools des composés suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de préférence de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

Comme deuxième tensioactif anionique, on utilise de préférence les alkylsulfates, les alkyléthersulfates et leurs mélanges, en particulier sous forme de sels de métaux alcalins (de préférence de sodium) ou alcalino-terreux, d'ammonium, d'amine ou d'aminoalcool.

La composition selon l'invention comprend préférentiellement au moins 0,5 % en poids de deuxième(s) tensioactif(s) anionique(s) par rapport au poids total de la composition. De préférence, elle comprend de 0,5 à 20 % en poids de deuxième(s) tensioactif(s) anionique(s), plus préférentiellement de 1 à 20 % en poids et encore plus préférentiellement de 3 à 15 % en poids, par rapport au poids total de la composition.

En outre, la composition selon l'invention comprend au moins un tensioactif amphotère ou zwittérionique.

Les tensioactifs amphotères ou zwittérioniques utilisables dans la présente invention peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (II) et (III) :

Rₐ-CONHCH₂CH₂-N(R_{b})(R_{c})(CH₂COO⁻) (II)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH₂CH₂-N(B)(B') (III)
dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

La composition selon l'invention comprend préférentiellement au moins 0,1 % en poids de tensioactif(s) amphotère(s) ou zwittérionique(s) par rapport au poids total de la composition. De préférence, elle comprend de 0,1 à 10 % en poids de tensioactif(s) amphotère(s) ou zwittérionique(s), plus préférentiellement de 0,5 à 8 % en poids et encore plus préférentiellement de 1 à 8 % en poids, par rapport au poids total de la composition.

La composition objet de la présente invention comprend au moins un ester de sorbitan oxyéthyléné.

Les composés utilisables à cet effet comprennent notamment les dérivés oxyéthylénés des mono et des polyesters d'acides gras en C₈₋₃₀ et du sorbitan, ayant de 1 à 50 motifs d'oxyde d'éthylène. On utilise de préférence les dérivés oxyéthylénés des mono et des polyesters d'acides gras en C₁₂₋₂₄ et du sorbitan, ayant de 4 à 20 motifs d'oxyde d'éthylène.

De tels composés sont également connus sous le nom de polysorbates. Ils sont entre autres commercialisés sous la dénomination TWEEN par la société UNIQEMA. Citons par exemple : le mono-laurate de sorbitan oxyéthylène à 4OE, commercialisé sous la dénomination TWEEN 21, le mono-laurate de sorbitan oxyéthylène à 20 OE, commercialisé sous la dénomination TWEEN 20, le mono- palmitate de sorbitan oxyéthylène à 20 OE commercialisé sous la dénomination TWEEN 40, le mono-stéarate de sorbitan oxyéthylène à 20 OE commercialisé sous la dénomination TWEEN 60, le mono-stéarate de sorbitan oxyéthylène à 4 OE commercialisé sous la dénomination TWEEN 61, le tri-stéarate de sorbitan oxyéthylène à 20 OE commercialisé sous la dénomination TWEEN 65, le mono-oléate de sorbitan oxyéthylène à 20 OE commercialisé sous la dénomination TWEEN 80, le mono-oléate de sorbitan oxyéthylène à 5 OE commercialisé sous la dénomination TWEEN 81, le tri-oléate de sorbitan oxyéthylène à 20 OE commercialisé sous la dénomination TWEEN 85.

Dans le présent exposé, et de manière bien connue en soi, on désigne par « composé à X OE » un composé oxyéthyléné comprenant X motifs oxyéthylène par molécule.

De préférence, l'acide gras de l'ester de sorbitan oxyéthyléné est un acide gras saturé.

Les esters de sorbitan préférés sont le mono-laurate de sorbitan oxyéthylène à 4 OE, le mono-laurate de sorbitan oxyéthylène à 20 OE, et leurs mélanges.

Selon un mode de réalisation préféré, la composition selon l'invention comprend un mélange de mono-laurate de sorbitan oxyéthylène à 4 OE et de mono-laurate de sorbitan oxyéthylène à 20 OE.

La composition selon l'invention comprend avantageusement au moins 0,5 % en poids d'ester de sorbitan oxyéthyléné par rapport au poids total de la composition. De préférence, elle comprend de 0,5 à 10 % en poids d'ester de sorbitan oxyéthyléné, plus préférentiellement de 2 à 9 % en poids, et encore plus préférentiellement de 4 à 8 % en poids, par rapport au poids total de la composition.

La composition selon l'invention comprend enfin au moins un polymère cationique, et/ou au moins un polymère amphotère ou zwitterionique.

Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont une masse moléculaire moyenne en poids supérieure à 10⁵, de préférence supérieure à 10⁶ et mieux encore comprise entre 10⁶ et 10⁸.

Parmi les polymères cationiques, on peut citer plus particulièrement les polymères du type polyamine, polyaminoamide et polyammonium quaternaire. Ils sont notamment décrits dans les brevets français nos 2 505 348 et 2 542 997. Parmi ces polymères, on peut citer :
(1) les homopolymères ou copolymères dérivés d'esters ou d'amides acryliques ou méthacryliques et comportant au moins un des motifs de formules suivantes: 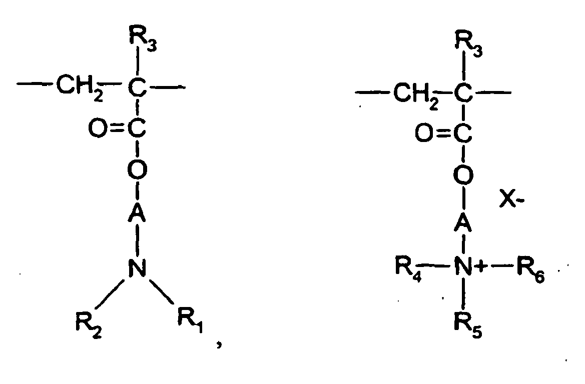 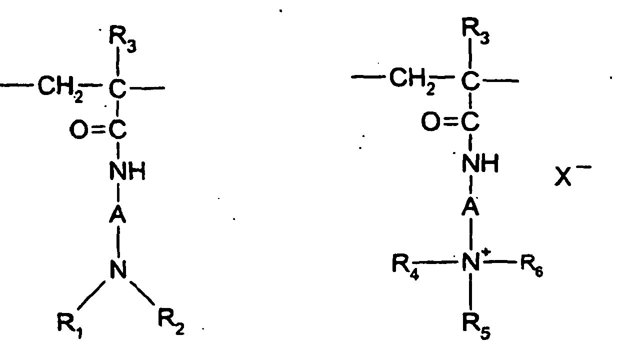 dans lesquelles:
   R₁ et R₂, identiques ou différents, représentent un atome d'hydrogène ou un groupe alkyle ayant de 1 à 6 atomes de carbone, et de préférence un groupe méthyle ou éthyle ;
   R₃, identiques ou différents, désignent un atome d'hydrogène ou un groupe CH₃ ;
   les symboles A, identiques ou différents, représentent un groupe alkyle, linéaire ou ramifié, comportant de 1 à 6 atomes de carbone, de préférence 2 ou 3 atomes de carbone, ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle, et de préférence un groupe alkyle ayant de 1 à 6 atomes de carbone ;
   X désigne un anion dérivé d'un acide minéral ou organique tel qu'un anion méthosulfate ou un halogénure comme le chlorure ou le bromure.

Les copolymères de la famille (1) peuvent contenir en outre un ou plusieurs motifs dérivant de comonomères pouvant être choisis dans la famille des acrylamides, méthacrylamides, diacétone-acrylamides, acrylamides et méthacrylamides substitués sur l'atome d'azote par des groupes alkyle inférieur (C₁-C₄), des groupes dérivés des acides acryliques ou méthacryliques ou de leurs esters, de vinyllactames tels que la vinylpyrrolidone ou le vinylcaprolactame, d'esters vinyliques.

Ainsi, parmi ces copolymères de la famille (1), on peut citer :
- les copolymères d'acrylamide et de méthacrylate de diméthylaminoéthyle quaternisé au sulfate de diméthyle ou avec un halogénure de diméthyle,
- les copolymères d'acrylamide et de chlorure de méthacryloyloxyéthyltriméthylammonium décrits, par exemple, dans la demande de brevet EP-A-080976,
- les copolymères d'acrylamide et de méthosulfate de méthacryloyloxyéthyltriméthylammonium,
- les copolymères vinylpyrrolidone/acrylate ou méthacrylate de dialkylaminoalkyle quaternisés ou non. Ces polymères sont décrits en détail dans les brevets français 2 077 143 et 2 393 573,
- les terpolymères méthacrylate de diméthylaminoéthyle/vinylcaprolactame/vinylpyrrolidone,
- les copolymères vinylpyrrolidone/méthacrylamidopropyl-diméthylamine, et
- les copolymères vinylpyrrolidone/méthacrylamide de diméthylaminopropyle quaternisé.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammoniums quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium, de diméthyldiallylammonium.
   Les produits commercialisés répondant à cette définition sont plus particulièrement les produits vendus sous la dénomination "Celquat^{®} L 200" et "Celquat^{®} H 100" par la Société National Starch.
(4) Les polysaccharides cationiques non cellulosiques décrits dans les brevets US 3 589 578 et 4 031 307 tels que les gommes de guar contenant des groupements cationiques trialkylammonium. On utilise, par exemple, des gommes de guar modifiées par un sel, par exemple le chlorure, de 2,3-époxypropyltriméthylammonium.
   De tels produits sont commercialisés notamment sous les dénominations commerciales de JAGUAR^{®} C13 S, JAGUAR^{®} C15, JAGUAR^{®} C17 ou JAGUAR^{®} C162 par la société MEYHALL.
(5) Les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361.
(6) Les polyaminoamides solubles dans l'eau, préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alkylés ou s'ils comportent une ou plusieurs fonctions amines tertiaires, quaternisées. De tels polymères sont notamment décrits dans les brevets français 2 252 840 et 2 368 508.
(7) Les dérivés de polyaminoamides résultant de la condensation de polyalkylènes-polyamines avec des acides polycarboxyliques, suivie d'une alkylation par des agents bifonctionnels. On peut citer, par exemple, les polymères acide adipique/diakylaminohydroxyalkyl-dialkylènetriamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
   Parmi ces dérivés, on peut citer plus particulièrement les polymères acide adipique/diméthylaminohydroxypropyl-diéthylènetriamine.
(8) Les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347.
(9) Les cyclopolymères d'alkyldiallylamine ou de dialkyldiallylammonium tels que les homopolymères ou copolymères comportant, comme constituant principal de la chaîne, des motifs répondant aux formules (Va) ou (Vb) : 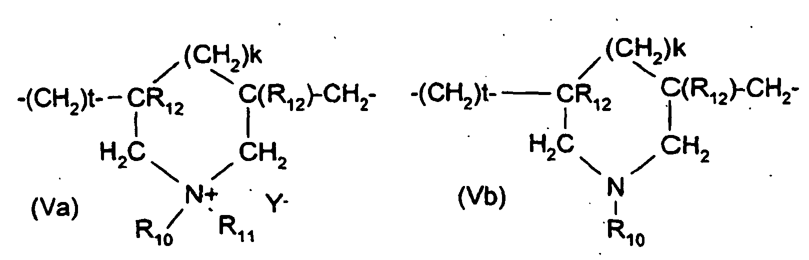 dans lesquelles k et t sont égaux à 0 ou 1, la somme k + t étant égale à 1 ; R₁₂ désigne un atome d'hydrogène ou un groupe méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou alors R₁₀ et R₁₁ peuvent désigner conjointement avec l'atome d'azote auquel ils sont rattachés, des groupements hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2 080 759 et dans son certificat d'addition 2 190 406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "MERQUAT^{®} 100" par la société CALGON (et ses homologues de faibles masses moléculaires moyenne en poids) et les copolymères de chlorure de diallyldiméthylammonium et d'acrylamide commercialisés sous la dénomination "MERQUAT^{®} 550".
(10) Les polymères de diammonium quaternaire contenant des motifs récurrents répondant à la formule (VI) : 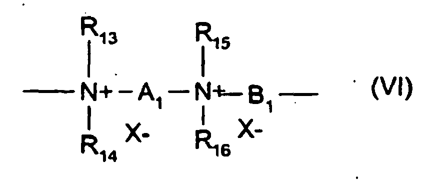 dans laquelle :
   R₁₃, R₁₄, R₁₅ et R₁₆, identiques ou différents, représentent des groupes aliphatiques, alicycliques ou arylaliphatiques contenant de 1 à 20 atomes de carbone ou des groupes hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote, ou bien R₁₃, R₁₄, R₁₅ et R₁₆ représentent un groupe alkyle en C₁-C₆, linéaire ou ramifié, substitué par un groupement nitrile, ester, acyle, amide ou -CO-O-R₁₇-E ou -CO-NH-R₁₇-E où R₁₇ est un groupe alkylène et E un groupement ammonium quaternaire ;
   A₁ et B₁ représentent des groupements polyméthyléniques contenant de 2 à 20 atomes de carbone, pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- désigne un anion dérivé d'un acide minéral ou organique;
   A₁, R₁₃ et R₁₅ peuvent former avec les deux atomes d'azote auxquels ils sont rattachés un cycle pipérazinique ; en outre, si A₁ désigne un groupe alkylène ou hydroxyalkylène linéaire ou ramifié, saturé ou insaturé, B₁ peut également désigner un groupement :

      -(CH₂)ₙ-CO-E'-OC-(CH₂)ₙ-
   dans lequel E' désigne :
   a) un reste de glycol de formule -O-Z-O-, où Z désigne un groupe hydrocarboné linéaire ou ramifié, ou un groupement répondant à l'une des formules suivantes :

      -(CH₂-CH₂-O)ₓ-CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule -NH-Y-NH-, où Y désigne un groupe hydrocarboné linéaire ou ramifié, ou bien le groupe divalent -CH₂-CH₂-S-S-CH₂-CH₂- ;
   d) un groupement uréylène de formule -NH-CO-NH- .
   De préférence, X- est un anion tel que le chlorure ou le bromure.
   Des polymères de ce type sont notamment décrits dans les brevets français 2 320 330, 2 270 846, 2 316 271, 2 336 434 et 2 413 907 et les brevets US 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020.
   On peut utiliser plus particulièrement les polymères qui sont constitués de motifs récurrents répondant à la formule : 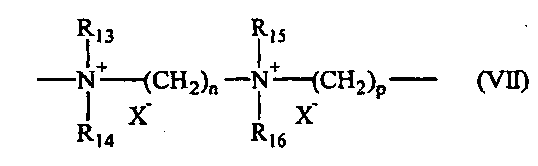 dans laquelle R₁₃, R₁₄, R₁₅ et R₁₆, identiques ou différents, désignent un groupe alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (VIII) : 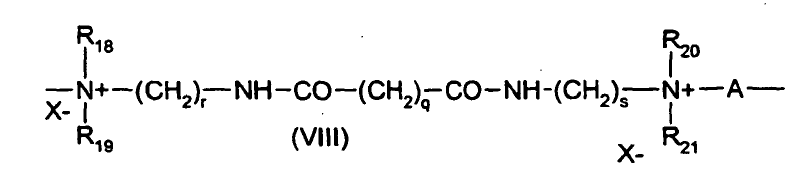 dans laquelle :
   R₁₈, R₁₉, R₂₀ et R₂₁, identiques ou différents, représentent un atome d'hydrogène ou un groupe méthyle, éthyle, propyle, β-hydroxyéthyle, β-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X- désigne un anion tel qu'un halogénure,
   A désigne un radical d'un dihalogénure ou représente de préférence -CH₂-CH₂-O-CH₂-CH₂-.
   De tels composés sont notamment décrits dans la demande de brevet EP-A-122 324.
(12) Les polymères quaternaires de vinylpyrrolidone et de vinylimidazole.
(13) Les polymères réticulés de sels de méthacryloyloxyalkyl(C₁-C₄) trialkyl(C₁-C₄)ammonium tels que les polymères obtenus par homopolymérisation du méthacrylate de diméthylaminoéthyle quaternisé par le chlorure de méthyle, ou par copolymérisation de l'acrylamide avec le méthacrylate de diméthylaminoéthyle quaternisé par le chlorure de méthyle, l'homopolymérisation ou la copolymérisation étant suivie d'une réticulation par un composé à insaturation oléfinique, en particulier le méthylène-bisacrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose comportant des groupements ammonium quaternaires tels que les produits vendus sous la dénomination "JR 400" par la Société UNION CARBIDE CORPORATION, les cyclopolymères cationiques, en particulier les homopolymères ou copolymères de chlorure de diméthyldiallylammonium, vendus sous les dénominations MERQUAT^{®} 100, MERQUAT^{®} 550 et MERQUAT^{®} S par la société CALGON, les gommes de guar modifiées par un sel de 2,3-époxypropyl-triméthylammonium, les polymères quaternaires de vinylpyrrolidone et de vinylimidazole.

Lorsque la composition selon l'invention comprend un ou plusieurs polymère (s) cationique(s), elle comprend préférentiellement au moins 0,01 % en poids de tel(s) polymère(s) par rapport au poids total de la composition. De préférence, elle comprend de 0,05 à 10 % en poids de polymère(s) cationique(s), plus préférentiellement de 0,1 à 5 % en poids, et encore plus préférentiellement de 0,1 à 2% en poids, par rapport au poids total de la composition.

Les polymères amphotères ou zwittérioniques utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
   Le composé vinylique peut être également un sel de dialkyldiallylammonium tel que le sel (par exemple chlorure) de diméthyldiallylammonium. Les copolymères d'acide acrylique et de ce dernier monomère sont proposés sous les appellations MERQUAT 280, MERQUAT 295 par la société NALCO.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethylmethacrylate copolymer.
(3) Les polyaminoamides réticulés et alcoylés partiellement ou totalement dérivant de polyaminoamides de formule générale : 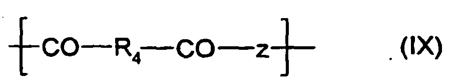 dans laquelle R₄ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 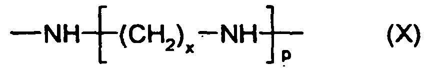 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (X) ci-dessus, dans lequel x=2 et p=1 et qui dérive de l'éthylènediamine, ou le radical dérivant de la pipérazine : 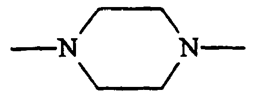
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 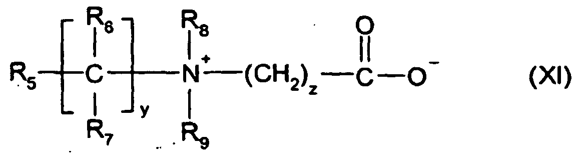 dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle/méthacrylate de diméthyl-carboxyméthylammonio-éthyl.
(5) Les polymères dérivés du chitosane comportant des motifs monomères répondant aux formules (XII), (XIII) et (XIV) suivantes : 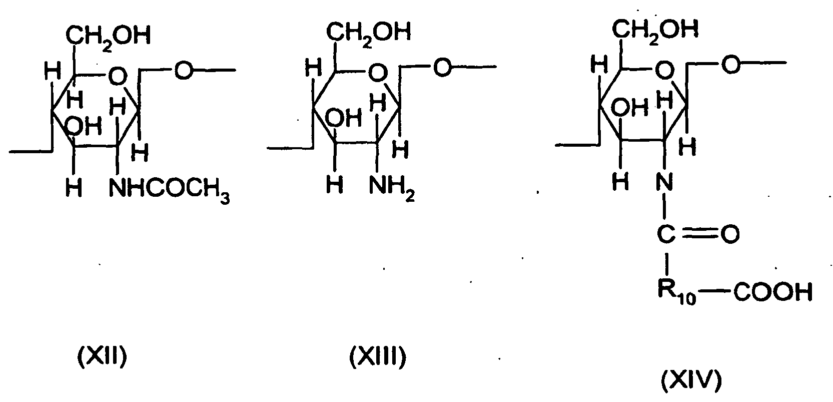 le motif (XII) étant présent dans des proportions comprises entre 0 et 30%, le motif (XIII) dans des proportions comprises entre 5 et 50% et le motif (XIV) dans des proportions comprises entre 30 et 90%, étant entendu que dans ce motif (XIV), R₁₀ représente un radical de formule : 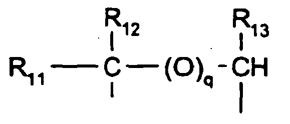 dans laquelle
   si q=0, R₁₁, R₁₂ et R₁₃, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₁, R₁₂ et R₁₃ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XV) tels que ceux décrits par exemple dans le brevet français 1 400 366 : 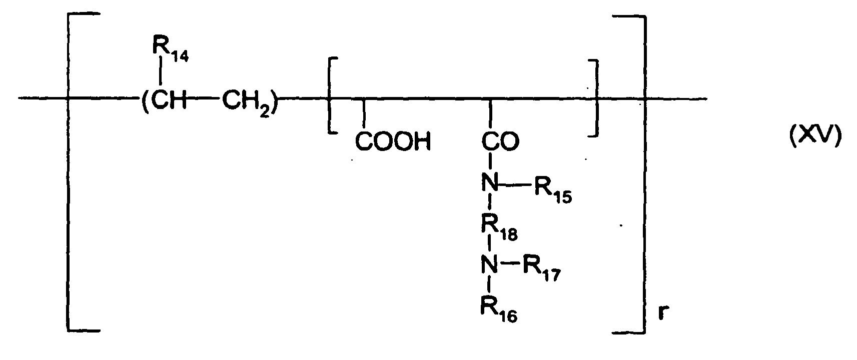 dans laquelle R₁₄ représente un atome d'hydrogène, un radical CH₃O, CH₃CH₂O, phényle, R₁₅ désigne l'hydrogène ou un radical alkyle inférieur tel que méthyle, éthyle, R₁₆ désigne l'hydrogène ou un radical alkyle inférieur tel que méthyle, éthyle, R₁₇ désigne un radical alkyle inférieur tel que méthyle, éthyle ou un radical répondant à la formule : -R₁₈-N(R₁₆)₂, R₁₈ représentant un groupement -CH₂-CH₂- , -CH₂-CH₂-CH₂- , -CH₂-CH(CH₃)- , R₁₆ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D-

      où D désigne un radical 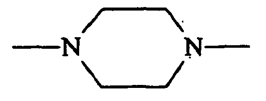 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X-

      où D désigne un radical 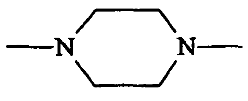 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Lorsque la composition selon l'invention comprend un ou plusieurs polymère(s) amphotère(s) ou zwittérionique(s), elle comprend avantageusement au moins 0,01 % en poids de tel(s) polymère(s) par rapport au poids total de la composition. De préférence, elle comprend de 0,05 à 10 % en poids de polymère(s) amphotère(s) ou zwittérionique(s) et plus préférentiellement de 0,1 à 5 % en poids, encore plus préférentiellement de 0,1 à 2 % en poids, par rapport au poids total de la composition.

Selon un mode de réalisation préféré, la composition selon l'invention comprend au moins deux polymères différents, choisis parmi les polymères cationiques, les polymères amphotères et les polymères zwittérioniques.

Dans un autre mode de réalisation préféré, la composition comprend au moins un polymère cationique et au moins un polymère amphotère ou zwittérionique.

La composition selon l'invention peut également comprendre, en plus du (des) ester(s) de sorbitan oxyéthyléné(s) un ou plusieurs autres tensioactifs non-ioniques additionnels, différents de ces derniers.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels est de préférence comprise dans l'intervalle allant de 0,01 à 10 % en poids, mieux encore de 0,05 à 5% en poids par rapport au poids total de la composition.

La composition selon l'invention présente de préférence une teneur totale en tensioactifs anioniques, non-ioniques, amphotères et zwittérioniques comprise dans l'intervalle allant de 4 à 50 % en poids, mieux encore de 4 à 20 % en poids, par rapport au poids total de la composition.

Les compositions selon la présente invention peuvent comprendre en outre au moins un tensioactif cationique.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,01 à 10 % en poids, mieux encore de 0,05 à 5 % en poids, et encore plus préférentiellement de 0,3 à 3 % en poids par rapport au poids total de la composition cosmétique.

La composition selon l'invention peut comprendre en outre au moins une silicone.

Les silicones utilisables conformément à l'invention peuvent être solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les organopolysiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 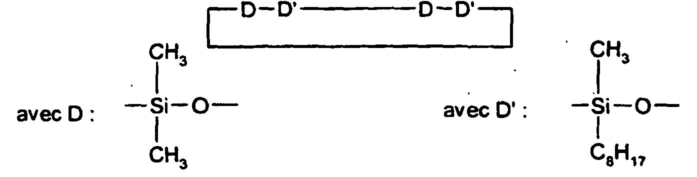 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyldiphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane,
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Peuvent également être employés des mélanges de silicones tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements ammonium quaternaires comme les produits commercialisés sous les dénominations ABILQUAT 3272 et ABILQUAT 3474 par la société GOLDSCHMIDT ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

Les silicones particulièrement préférées dans l'invention sont les polydiméthylsiloxanes tels que les polydiméthylsiloxanes à groupements terminaux triméthylsilyle, ou les polydiméthylsiloxanes à groupements terminaux hydroxydiméthylsilyle, et les silicones aminées.

Lorsque lesdites silicones sont présentes, elles sont contenues de préférence en une quantité allant de 0,05 à 20 % en poids, plus particulièrement de 0,1 à 10 % en poids, et mieux encore de 0,5 à 5 % en poids par rapport au poids total de la composition.

Les compositions selon l'invention peuvent se présenter sous la forme de gels douches, de shampooings, de compositions à appliquer avant ou après un shampooing, ces dernières se présentant sous la forme d'une lotion plus ou moins épaissie, d'un gel ou d'une émulsion.

Selon un mode de réalisation, la composition comprend en outre au moins un agent antipelliculaire. Comme agents antipelliculaires, peuvent être employés par exemple des composés tels que la piroctone olamine, la pyrithione de zinc, l'acide salicylique, le disulfure de sélénium, et leurs mélanges.

La composition comprend alors de 0,001 à 10 % en poids d'agent(s) antipelliculaire(s), de préférence de 0,1 à 5 % en poids, encore plus préférentiellement de 0,2 à 2 % en poids, par rapport au poids total de la composition.

Le milieu aqueux est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH de la composition selon l'invention est généralement inférieur à 8,5, et de préférence compris dans l'intervalle allant de 4 à 7.

La composition selon l'invention peut comprendre en outre un ou plusieurs additifs classiques bien connus dans la technique, tels que les agents antichute, les agents oxydants, les céramides et pseudo-céramides, les vitamines et pro-vitamines dont le panthénol, les huiles végétales, animales, minérales ou synthétiques, les cires, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

Comme agents opacifiants, peuvent être employés par exemple des composés tels que le distéarate d'éthylène glycol.

L'homme de métier veillera à choisir les éventuels additifs et leurs quantités de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

La composition selon l'invention peut être utilisée notamment comme composition de traitement ou de soin cosmétique des cheveux ou de la peau. En particulier, elle peut être utilisée comme shampooing ou composition à appliquer avant ou après un shampooing.

Un autre objet de l'invention est un procédé de traitement cosmétique, qui comprend l'application sur les cheveux d'une quantité efficace d'une composition cosmétique telle que décrite ci-dessus.

Selon un mode de mise en oeuvre préféré, un tel procédé consiste à appliquer sur les cheveux une quantité efficace de la composition cosmétique, à éventuellement rincer après un éventuel temps de pause.

Lorsque l'on applique la composition selon l'invention sous forme d'une lotion ou d'une crème avant ou après shampooing, on la laisse éventuellement pauser sur les cheveux pendant environ 1/2 minute à 5 minutes, puis on rince éventuellement à l'eau.

Les exemples suivants sont donnés à titre illustratif de la présente invention, et ne sauraient en limiter la portée.

### EXEMPLES

Dans les exemples suivants, toutes les quantités sont indiquées en pour cent en poids de matière active par rapport au poids total de la composition, sauf indication contraire.

### EXEMPLE 1 :

Deux compositions de shampooing, conformes à l'invention, sont préparées à partir des ingrédients indiqués dans le tableau ci-dessous.

La composition A est un shampooing antipelliculaire, tandis que la composition B est un shampooing pour enfants.

| Composition | A | B |
|---|---|---|
| Lauryl éther sulfate de sodium à 2,2 OE en solution aqueuse (Texapon N702 de Cognis) | 9,9 | 7 |
| Cocoyl amidopropyl bétaine / monolaurate de glycérine en solution aqueuse 25 / 75 (Tégobétaine HS de Goldschmidt) | 3,9 | - |
| Mono-laurate de sorbitane oxyéthylène à 4 OE (Tween 21 de Uniqema) | 6 | 7 |
| Lauryl éther 4 OE sulfate de sodium / magnésium 80/20 en solution aqueuse à 52% (Texapon ASV 50 de Cognis) | 3,1 | 2,5 |
| Monolaurate de sorbitan oxyéthylène à 20 OE (Tween 20 de Uniqema) | 2 | 0,5 |
| Distéarate d'éthylène glycol (Tegin BL 315 de Goldschmidt) | 1,5 | 1,5 |
| N-cocoyl amidoéthyl, N-éthoxycarboxyméthyl glycinate de sodium (Miranol C2M conc de Rhodia) | 0,6 | 3,1 |
| Hexylène glycol (2 méthyl-2,4 pentanediol) | 1 | 0,5 |
| Copolymère méthacryloyléthyl N,N-diméthyl carboxyméthyl bétaïne / méthacrylate de méthyle à 22% dans eau / éthanol 68/32 (Mexomere PX de Chimex) | 0,2 | 0,15 |
| Dioléate de polyéthylène glycol (55 OE) et de propylène glycol en solution hydroglycolique (NTIL 141 liquid de Goldschmidt) | 0,8 | |
| 1-hydroxy-4-méthyl-6-triméthylpentyl-2-pyridone, sel de monoéthanolamine (Octopirox de Clariant) | 0,5 | |
| Benzoate de sodium | 0,5 | |
| Parfum | 0,5 | 0,5 |
| Mélange de p-hydroxybenzoate de méthyle, butyle, éthyle, propyle, isobutyle (7/57/22/14) (Nipastat de Nipa) | 0,5 | |
| Chlorure d'hydroxypropyl guar triméthylammonium (Jaguar C13S de Rhodia) | 0,25 | 0,17 |
| acide salicylique en poudre | 0,2 | |
| Polymère carboxyvinylique synthétisé dans un mélange acétate d'éthyle / cyclohexane (Carbopol 980 de Noveon) | 0,1 | 0,1 |
| p-hydroxybenzoate de méthyle | 0,02 | 0,02 |
| Diméthylol-1,3 diméthyl-5,5 hydantoine en solution aqueuse (Glydant LTD de Lonza) | | 0,14 |
| p-hydroxybenzoate de méthyle, sel de sodium | | 0,18 |
| (Nipagin M sodium de Clariant) | | |
| Propylène glycol | 0,1 | 0,1 |
| Mélange de glycérides de palme oxyéthyléné (200 OE) et de coprah oxyéthyléné (7 OE) en suspension aqueuse (Rewoderm LI-S80 de Goldschmidt) | | 1,20 |
| Eau qsp | 100% | 100% |

Les compositions A et B conformes à l'invention se sont avérées présenter une excellente tolérance vis-à-vis du cuir chevelu. En particulier, on a observé très peu de réactions d'inconfort.

En outre, ces compositions, et plus particulièrement la composition B, sont extrêmement douces et présentent une excellente tolérance oculaire.

Enfin, ces compositions présentent de remarquables propriétés cosmétiques de douceur, de démêlage, de lissage des cheveux, de coiffant et de volume.

### EXEMPLE 2 : Essais comparatifs

Une composition de shampooing C, selon l'invention, et une composition de shampooing D, ne comprenant pas de tensioactifs sous forme de sels de magnésium, sont préparées à partir des ingrédients indiqués dans le tableau ci-dessous.

| Composition | C | D |
|---|---|---|
| Lauryl éther sulfate de sodium à 2,2 OE en solution aqueuse (Texapon N702 de Cognis) | 7 | 10,5 |
| Lauryl éther 4 OE sulfate de sodium / magnésium 80/20 en solution aqueuse à 52% (Texapon ASV 50 de Cognis) | 3,5 | |
| N-cocoyl amidoéthyl, N-éthoxycarboxyméthyl glycinate de sodium (Miranol C2M conc de Rhodia) | 2,01 | 2,01 |
| Mono-laurate de sorbitane oxyéthylène à 4 OE (Tween 21 de Uniqema) | 8 | 8 |
| Chlorure d'hydroxypropyl guar triméthylammonium (Jaguar C 13 S de Rhodia) | 0,25 | 0,25 |
| Copolymère méthacryloyléthyl N,N-diméthyl carboxyméthyl bétaïne / méthacrylate de méthyle à 22% dans eau / éthanol 68/32 (Mexomere PX de Chimex) | 0,11 | 0,11 |
| Acide salicylique en poudre | 0,2 | 0,2 |
| Conservateurs | 1 | 1 |
| Acide citrique q.s. | pH 5,5 | pH 5,5 |
| Eau qsp | 100% | 100% |

Les compositions de shampooing C et D ont été testées chacune sur 10 modèles. Les résultats obtenus sont les suivants :
pour 90% des modèles testés, la composition C apporte un meilleur lissage visuel par rapport à la composition D, et pour 70% des modèles testés, les cheveux traités avec la composition C sont plus souples et ont un meilleur toucher par rapport à ceux traité avec la composition D.

Ainsi, la composition C conforme à l'invention s'est avérée procurer de meilleures propriétés cosmétiques par rapport à la composition D. A quantité totale de tensioactif anionique égale par ailleurs, la présence d'un tensioactif anionique sous forme de sel de magnésium améliore de manière substantielle les propriétés de la composition de shampooing.

## Revendications

1. Composition cosmétique pour le lavage des matières kératiniques comprenant, dans un milieu aqueux :
(a) au moins un premier tensioactif anionique sous forme de sel de magnésium,
(b) au moins un deuxième tensioactif anionique différent du premier et qui n'est pas sous forme de sel de magnésium,
(c) au moins un tensioactif amphotère ou zwittérionique,
(d) au moins un ester de sorbitan oxyéthyléné, et
(e) au moins un polymère cationique et/ou au moins un polymère amphotère ou zwittérionique.

2. Composition selon la revendication précédente, **caractérisée en ce que** ledit premier tensioactif anionique est choisi parmi les sels de magnésium des composés suivants: les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates, les acylglutamates, et leurs mélanges, les groupes alkyle et acyle de tous ces composés comportant de 6 à 24 atomes de carbone et le groupe aryle désignant de préférence un groupe phényle ou benzyle.

3. Composition selon la revendication précédente, **caractérisée en ce que** ledit premier tensioactif anionique comprend au moins un alkyléthersulfate de magnésium contenant de 1 à 16 motifs d'oxyde d'éthylène.

4. Composition selon l'une quelconque des revendications précédentes, **caractérisée en ce qu'**elle comprend au moins 0,1 % en poids dudit premier tensioactif anionique par rapport au poids total de la composition.

5. Composition selon la revendication précédente, **caractérisée en ce qu'**elle comprend de 0,1 à 10 % en poids dudit premier tensioactif anionique, plus préférentiellement de 0,2 à 8 % en poids et encore plus préférentiellement de 0,3 à 2 % en poids, par rapport au poids total de la composition.

6. Composition selon l'une quelconque des revendications précédentes, **caractérisée en ce que** le deuxième tensioactif anionique est choisi parmi les sels, en particulier les sels de métaux alcalins ou alcalino-terreux autres que le magnésium, tels que les sels de sodium, les sels d'ammonium, les sels d'amines, les sels d'aminoalcools des composés suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

7. Composition selon l'une quelconque des revendications précédentes, **caractérisée en ce que** le deuxième tensioactif anionique est choisi parmi les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de préférence de 12 à 20 atomes de carbone.

8. Composition selon l'une quelconque des revendications précédentes, **caractérisée en ce que** le deuxième tensioactif anionique et choisi parmi les acyl-lactylates dont le groupe acyle comporte de 8 à 20 atomes de carbone, les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

9. Composition selon la revendication 6, **caractérisée en ce que** le deuxième tensioactif anionique est choisi parmi les alkylsulfates, les alkyléthersulfates et leurs mélanges, en particulier sous forme de sels de métaux alcalins ou alcalino-terreux, d'ammonium, d'amine ou d'aminoalcool.

10. Composition selon l'une quelconque des revendications précédentes, **caractérisée en ce qu'**elle comprend au moins 0,5 % en poids de deuxième(s) tensioactif(s) anionique(s) par rapport au poids total de la composition.

11. Composition selon la revendication 10, **caractérisée en ce qu'**elle comprend de 0,5 à 20 % en poids de deuxième(s) tensioactif(s) anionique(s), plus préférentiellement de 1 à 20 % en poids et encore plus préférentiellement de 3 à 15 % en poids, par rapport au poids total de la composition.

12. Composition selon l'une quelconque des revendications précédentes, **caractérisée en ce que** le tensioactif amphotère ou zwittérionique est choisi parmi :
- les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate, et
- les alkyl(C₈₋₂₀)bétaïnes, les sulfobétaïnes, les (alkyl en C₈-₂₀)amido(alkyl en C₆₋₈)bétaïnes ou les (alkyl en C₈₋₂₀)amido(alkyl en C₆₋₈)sulfobétaïnes.

13. Composition selon la revendication 12, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆-₈)bétaïnes et leurs mélanges.

14. Composition selon l'une quelconque des revendications précédentes, **caractérisée en ce qu'**elle comprend au moins 0,1 % en poids de tensioactif(s) amphotère(s) ou zwittérionique(s) par rapport au poids total de la composition.

15. Composition selon la revendication 14, **caractérisée en ce qu'**elle comprend de 0,1 à 10 % en poids de tensioactif(s) amphotère(s) ou zwittérionique(s), plus préférentiellement de 0,5 à 8 % en poids et encore plus préférentiellement de 1 à 8 % en poids, par rapport au poids total de la composition.

16. Composition selon l'une quelconque des revendications précédentes, **caractérisée en ce que** l'ester de sorbitan oxyéthyléné est choisi parmi les dérivés oxyéthylénés des mono et des polyesters d'acides gras en C₈₋₃₀ et du sorbitan, ayant de 1 à 50 motifs d'oxyde d'éthylène.

17. Composition selon la revendication précédente, **caractérisée en ce que** l'ester de sorbitan oxyéthyléné est choisi parmi les dérivés oxyéthylénés des mono et des polyesters d'acides gras en C₁₂₋₂₄ et du sorbitan, ayant de 4 à 20 motifs d'oxyde d'éthylène.

18. Composition selon la revendication précédente, **caractérisée en ce que** l'acide gras de l'ester de sorbitan oxyéthyléné est un acide gras saturé.

19. Composition selon la revendication précédente, **caractérisée en ce que** l'ester de sorbitan oxyéthyléné est choisi parmi le mono-laurate de sorbitan oxyéthylène à 4 OE, le mono-laurate de sorbitan oxyéthylène à 20 OE, et leurs mélanges.

20. Composition selon l'une quelconque des revendications précédentes, **caractérisée en ce qu'**elle comprend au moins 0,5 % en poids d'ester de sorbitan oxyéthyléné par rapport au poids total de la composition.

21. Composition selon la revendication précédente, **caractérisée en ce qu'**elle comprend de 0,5 à 10 % en poids d'ester de sorbitan oxyéthyléné, de préférence de 2 à 9 % en poids, par rapport au poids total de la composition.

22. Composition selon l'une quelconque des revendications précédentes, **caractérisée en ce que** le polymère cationique est choisi parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

23. Composition selon la revendication précédente, **caractérisée en ce que** le polymère cationique est choisi parmi les dérivés d'éther de cellulose comportant des groupements ammonium quaternaires, les cyclopolymères cationiques, en particulier les homopolymères ou copolymères de chlorure de diméthyldiallylammonium, les gommes de guar modifiées par un sel de 2,3-époxypropyl-triméthylammonium, les polymères quaternaires de vinylpyrrolidone et de vinylimidazole.

24. Composition selon l'une quelconque des revendications précédentes, **caractérisée en ce qu'**elle comprend au moins 0,01 % en poids de polymère(s) cationique(s) par rapport au poids total de la composition.

25. Composition selon la revendication précédente, **caractérisée en ce qu'**elle comprend de 0,05 à 10 % en poids de polymère(s) cationique(s), et plus préférentiellement de 0,1 à 5 % en poids, par rapport au poids total de la composition.

26. Composition selon l'une quelconque des revendications précédentes, **caractérisée en ce que** le polymère amphotère ou zwittérionique est choisi parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M désignent des groupements dérivant de monomères zwittérioniques de carboxybétaïnes ou de sulfobétaïnes, ou bien K et M désignent une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

27. Composition selon la revendication précédente, **caractérisée en ce que** le polymère amphotère ou zwittérionique est un polymère résultant de la copolymérisation d'un monomère dérivé d'un composé vinylique portant un groupement carboxylique tel que l'acide acrylique, l'acide méthacrylique, l'acide maléique, l'acide alpha-chloracrylique, et d'un monomère basique dérivé d'un composé vinylique substitué contenant au moins un atome basique.

28. Composition selon l'une quelconque des revendications précédentes, **caractérisée en ce qu'**elle comprend au moins 0,01 % en poids de polymères amphotères ou zwittérioniques par rapport au poids total de la composition.

29. Composition selon la revendication précédente, **caractérisée en ce qu'**elle comprend, de 0,05 à 10 % en poids de polymère(s) amphotère(s) ou zwittérionique(s) et plus préférentiellement de 0,1 à 5 % en poids, par rapport au poids total de la composition.

30. Composition selon l'une quelconque des revendications précédentes, **caractérisée en ce qu'**elle comprend au moins deux polymères différents, choisis parmi les polymères cationiques, les polymères amphotères et les polymères zwittérioniques.

31. Composition selon l'une quelconque des revendications précédentes, **caractérisée en ce qu'**elle comprend au moins un polymère cationique, et au moins un polymère amphotère ou zwittérionique.

32. Composition selon l'une quelconque des revendications précédentes, **caractérisée en ce qu'**elle comprend en outre au moins un agent antipelliculaire, tel que par exemple la piroctone olamine, la pyrithione de zinc, l'acide salicylique, le disulfure de sélénium, et leurs mélanges.

33. Composition selon la revendication précédente, **caractérisée en ce qu'**elle comprend de 0,001 à 10 % en poids d'agent(s) antipelliculaire(s), de préférence de 0,1 à 5 % en poids par rapport au poids total de la composition.

34. Composition selon l'une quelconque des revendications précédentes, **caractérisée en ce qu'**elle comprend en outre au moins une silicone.

35. Composition selon l'une quelconque des revendications précédentes, **caractérisée en ce que** le milieu aqueux est constitué d'eau ou d'un mélange d'eau et d'au moins un solvant cosmétiquement acceptable.

36. Composition selon la revendication 35, **caractérisée en ce que** le solvant est choisi parmi les alcools inférieurs en C₁₋C₄ et les polyols.

37. Composition selon l'une quelconque des revendications précédentes, **caractérisée en ce qu'**elle comprend en outre au moins un additif choisi parmi les agents antichute, les agents oxydants, les céramides et pseudo-céramides, les vitamines et pro-vitamines dont le panthénol, les huiles végétales, animales, minérales ou synthétiques, les cires, les filtres solaires, les pigments minéraux ou organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux ou organiques, les agents anti-oxydants, les hydroxyacides, les parfums et les agents conservateurs.

38. Utilisation de la composition selon l'une quelconque des revendications 1 à 37, comme composition de traitement ou de soin cosmétique des cheveux ou de la peau.

39. Utilisation de la composition selon l'une quelconque des revendications 1 à 37, comme shampooing ou composition à appliquer avant ou après un shampooing.

40. Procédé de traitement cosmétique, **caractérisé en ce qu'**il comprend l'application sur les cheveux d'une quantité efficace d'une composition selon l'une quelconque des revendications 1 à 37.

## Claims

1. Cosmetic composition for washing keratin materials, comprising, in an aqueous medium:
(a) at least a first anionic surfactant in the form of a magnesium salt,
(b) at least a second anionic surfactant different from the first and which is not in the form of a magnesium salt,
(c) at least one amphoteric or zwitterionic surfactant,
(d) at least one oxyethylenated sorbitan ester, and
(e) at least one cationic polymer and/or at least one amphoteric or zwitterionic polymer.

2. Composition according to the preceding claim, **characterized in that** said first anionic surfactant is chosen from magnesium salts of the following compounds: alkyl sulphates, alkyl ether sulphates, alkylamido ether sulphates, alkylaryl polyether sulphates, monoglyceride sulphates, alkyl sulphonates, alkylamide sulphonates, alkylaryl sulphonates, α-olefin sulphonates, paraffin sulphonates, alkylsulphosuccinates, alkyl ether sulphosuccinates, alkylamide sulphosuccinates, alkyl sulphoacetates, acylsarcosinates, acylglutamates, and mixtures thereof, the alkyl and acyl groups of all of these compounds containing from 6 to 24 carbon atoms and the aryl group preferably denoting a phenyl or benzyl group.

3. Composition according to the preceding claim, **characterized in that** said first anionic surfactant comprises at least one magnesium alkyl ether sulphate containing from 1 to 16 ethylene oxide units.

4. Composition according to any one of the preceding claims, **characterized in that** it comprises at least 0.1% by weight of said first anionic surfactant relative to the total weight of the composition.

5. Composition according to the preceding claim, **characterized in that** it comprises from 0.1 to 10% by weight of said first anionic surfactant, more preferably from 0.2 to 8% by weight, and even more preferably from 0.3 to 2% by weight, relative to the total weight of the composition.

6. Composition according to any one of the preceding claims, **characterized in that** the second anionic surfactant is chosen from salts, in particular salts of alkali metals or alkaline earth metals other than magnesium, such as sodium salts, ammonium salts, amine salts, or the aminoalcohol salts of the following compounds: alkyl sulphates, alkyl ether sulphates, alkylamido ether sulphates, alkylaryl polyether sulphates, monoglyceride sulphates, alkyl sulphonates, alkylamide sulphonates, alkylaryl sulphonates, α-olefin sulphonates, paraffin sulphonates, alkylsulphosuccinates, alkyl ether sulphosuccinates, alkylamide sulphosuccinates, alkyl sulphoacetates, acylsarcosinates and acylglutamates, the alkyl and acyl groups of all of these compounds containing from 6 to 24 carbon atoms and the aryl group preferably denoting a phenyl or benzyl group.

7. Composition according to any one of the preceding claims, **characterized in that** the second anionic surfactant is chosen from C₆₋₂₄ alkyl monoesters of polyglycoside dicarboxylic acids, such as alkyl glucoside citrates, alkyl polyglycoside tartrates and alkyl polyglycoside sulphosuccinates, alkyl sulphosuccinamates, acylisethionates and N-acyl taurates, the alkyl or acyl group of all of these compounds preferably containing from 12 to 20 carbon atoms.

8. Composition according to any one of the preceding claims, **characterized in that** the second anionic surfactant is chosen from acyl lactylates, the acyl group of which contains from 8 to 20 carbon atoms, alkyl-D-galactosiduronic acids and salts thereof, and also polyoxyalkylenated (C₆₋₂₄ alkyl) ether carboxylic acids, polyoxyalkylenated (C₆₋₂₄ alkyl) (C₆₋₂₄ aryl) ether carboxylic acids, polyoxyalkylenated (C₆₋₂₄ alkyl) amido ether carboxylic acids, and salts thereof, in particular those comprising from 2 to 50 ethylene oxide units, and mixtures thereof.

9. Composition according to Claim 6, **characterized in that** the second anionic surfactant is chosen from alkyl sulphates, alkyl ether sulphates and mixtures thereof, in particular in the form of alkali metal salts or alkaline earth metal salts, ammonium salts, amine salts or aminoalcohol salts.

10. Composition according to any one of the preceding claims, **characterized in that** it comprises at least 0.5% by weight of second anionic surfactant(s) relative to the total weight of the composition.

11. Composition according to Claim 10, **characterized in that** it comprises from 0.5 to 20% by weight of second anionic surfactant(s), more preferably from 1 to 20% by weight, and even more preferably from 3 to 15% by weight, relative to the total weight of the composition.

12. Composition according to any one of the preceding claims, **characterized in that** the amphoteric or zwitterionic surfactant is chosen from:
- derivatives of secondary or tertiary aliphatic amines in which the aliphatic group is a linear or branched chain comprising from 8 to 22 carbon atoms and containing at least one anionic group such as, for example, a carboxylate, sulphonate, sulphate, phosphate or phosphonate group, and
- (C₈₋₂₀) alkylbetaines, sulphobetaines, (C₈₋₂₀ alkyl) amido (C₆₋₈ alkyl) betaines or (C₈₋₂₀ alkyl)amido(C₆₋₈ alkyl)sulphobetaines.

13. Composition according to Claim 12, **characterized in that** the amphoteric or zwitterionic surfactant is chosen from (C₈₋₂₀ alkyl) betaines, (C₈₋₂₀ alkyl)amido(C₆₋₈ alkyl) betaines and mixtures thereof.

14. Composition according to any one of the preceding claims, **characterized in that** it comprises at least 0.1% by weight of amphoteric or zwitterionic surfactant(s) relative to the total weight of the composition.

15. Composition according to Claim 14, **characterized in that** it comprises from 0.1 to 10% by weight of amphoteric or zwitterionic surfactant(s), more preferably from 0.5 to 8% by weight, and even more preferably from 1 to 8% by weight, relative to the total weight of the composition.

16. Composition according to any one of the preceding claims, **characterized in that** the oxyethylenated sorbitan ester is chosen from oxyethylenated derivatives of monoesters and polyesters of C₈₋₃₀ fatty acids and of sorbitan, having from 1 to 50 ethylene oxide units.

17. Composition according to the preceding claim, **characterized in that** the oxyethylenated sorbitan ester is chosen from oxyethylenated derivatives of monoesters and polyesters of C₁₂₋₂₄ fatty acids and of sorbitan, having from 4 to 20 ethylene oxide units.

18. Composition according to the preceding claim, **characterized in that** the fatty acid of the oxyethylenated sorbitan ester is a saturated fatty acid.

19. Composition according to the preceding claim, **characterized in that** the oxyethylenated sorbitan ester is chosen from oxyethylene sorbitan monolaurate comprising 4 OE and oxyethylene sorbitan monolaurate comprising 20 OE, and mixtures thereof.

20. Composition according to any one of the preceding claims, **characterized in that** it comprises at least 0.5% by weight of oxyethylenated sorbitan ester relative to the total weight of the composition.

21. Composition according to the preceding claim, **characterized in that** it comprises from 0.5 to 10% by weight of oxyethylenated sorbitan ester, preferably from 2 to 9% by weight, relative to the total weight of the composition.

22. Composition according to any one of the preceding claims, **characterized in that** the cationic polymer is chosen from those which contain units comprising primary, secondary, tertiary and/or quaternary amine groups which can either be part of the main polymer chain, or can be carried by a side substituent directly connected to said chain.

23. Composition according to the preceding claim, **characterized in that** the cationic polymer is chosen from cellulose ether derivatives containing quaternary ammonium groups, cationic cyclopolymers, in particular dimethyldiallylammonium chloride homopolymers or copolymers, guar gums modified with a 2,3-epoxypropyltrimethylammonium salt, and quaternary polymers of vinylpyrrolidone and of vinylimidazole.

24. Composition according to any one of the preceding claims, **characterized in that** it comprises at least 0.01% by weight of cationic polymer(s) relative to the total weight of the composition.

25. Composition according to the preceding claim, **characterized in that** it comprises from 0.05 to 10% by weight of cationic polymer(s), and more preferably from 0.1 to 5% by weight, relative to the total weight of the composition.

26. Composition according to any one of the preceding claims, **characterized in that** the amphoteric or zwitterionic polymer is chosen from polymers containing K and M units distributed randomly in the polymer chain, where K denotes a unit derived from a monomer containing at least one basic nitrogen atom and M denotes a unit derived from an acidic monomer containing one or more carboxylic or sulphonic groups, or else K and M denote groups derived from zwitterionic monomers of carboxybetaines or of sulphobetaines, or else K and M denote a cationic polymer chain containing primary, secondary, tertiary or quaternary amine groups, in which at least one of the amine groups bears a carboxylic or sulphonic group connected via a hydrocarbon-based radical, or else K and M are part of a chain of a polymer comprising an α,β-dicarboxylic ethylene unit in which one of the carboxylic groups has been reacted with a polyamine containing one or more primary or secondary amine groups.

27. Composition according to the preceding claim, **characterized in that** the amphoteric or zwitterionic polymer is a polymer resulting from the copolymerization of a monomer derived from a vinyl compound bearing a carboxylic group, such as acrylic acid, methacrylic acid, maleic acid or alpha-chloroacrylic acid, and of a basic monomer derived from a substituted vinyl compound containing at least one basic atom.

28. Composition according to any one of the preceding claims, **characterized in that** it comprises at least 0.01% by weight of amphoteric or zwitterionic polymers relative to the total weight of the composition.

29. Composition according to the preceding claim, **characterized in that** it comprises from 0.05 to 10% by weight of amphoteric or zwitterionic polymer(s), and more preferably from 0.1 to 5% by weight, relative to the total weight of the composition.

30. Composition according to any one of the preceding claims, **characterized in that** it contains at least two different polymers, chosen from cationic polymers, amphoteric polymers and zwitterionic polymers.

31. Composition according to any one of the preceding claims, **characterized in that** it comprises at least one cationic polymer, and at least one amphoteric or zwitterionic polymer.

32. Composition according to any one of the preceding claims, **characterized in that** it also comprises at least one anti-dandruff agent, such as, for example, piroctone olamine, zinc pyrithione, salicylic acid and selenium disulphide, and mixtures thereof.

33. Composition according to the preceding claim, **characterized in that** it comprises from 0.001 to 10% by weight of anti-dandruff agent(s), preferably from 0.1 to 5% by weight relative to the total weight of the composition.

34. Composition according to any one of the preceding claims, **characterized in that** it also comprises at least one silicone.

35. Composition according to any one of the preceding claims, **characterized in that** the aqueous medium consists of water or of a mixture of water and at least one cosmetically acceptable solvent.

36. Composition according to Claim 35, **characterized in that** the solvent is chosen from C₁-C₄ lower alcohols and polyols.

37. Composition according to any one of the preceding claims, **characterized in that** it also comprises at least one additive chosen from anti-hairloss agents, oxidizing agents, ceramides and pseudoceramides, vitamins and provitamins, including panthenol, plant, animal, mineral or synthetic oils, waxes, sunscreens, coloured or non-coloured, inorganic or organic pigments, dyes, pearlescent agents and opacifiers, sequestrants, plasticizers, solubilizing agents, acidifying agents, basifying agents, inorganic or organic thickeners, antioxidants, hydroxy acids, fragrances and preserving agents.

38. Use of the composition according to any one of Claims 1 to 37, as a composition for the cosmetic treatment or care of the hair or of the skin.

39. Use of the composition according to any one of Claims 1 to 37, as a shampoo or a composition to be applied before or after a shampoo.

40. Cosmetic treatment process, **characterized in that** it comprises the application to the hair of an effective amount of a composition according to any one of Claims 1 to 37.

## Patentansprüche

1. Kosmetische Zusammensetzung für die Reinigung von Keratinsubstanzen, die in einem wässrigen Medium enthält:
(a) mindestens einen ersten anionischen grenzflächenaktiven Stoff in Form des Magnesiumsalzes,
(b) mindestens einen zweiten anionischen grenzflächenaktiven Stoff, der von dem ersten verschieden ist und nicht in Form des Magnesiumsalzes vorliegt,
(c) mindestens einen amphoteren oder zwitterionischen grenzflächenaktiven Stoff,
(d) mindestens einen ethoxylierten Sorbitanester, und
(e) mindestens ein kationisches Polymer und/oder mindestens ein amphoteres oder zwitterionisches Polymer.

2. Zusammensetzung nach dem vorherigen Anspruch, **dadurch gekennzeichnet, dass** der erste anionische grenzflächenaktive Stoff unter den Magnesiumsalzen der folgenden Verbindungen ausgewählt ist: Alkylsulfate, Alkylethersulfate, Alkylamidoethersulfate, Alkylarylpolyethersulfate, Monoglyceridsulfate, Alkylsulfonate, Alkylamidsulfonate, Alkylarylsulfonate, α-Olefinsulfonate, Paraffinsulfonate, Alkylsulfosuccinate, Alkylethersulfosuccinate, Alkylamidsulfosuccinate, Alkylsulfoacetate, Acylsarcosinate und Acylglutamate und deren Gemische, wobei die Alkyl- und Acylgruppe dieser Verbindungen 6 bis 24 Kohlenstoffatome enthält und die Arylgruppe vorzugsweise Phenyl oder Benzyl bedeutet.

3. Zusammensetzung nach dem vorherigen Anspruch, **dadurch gekennzeichnet, dass** der erste anionische grenzflächenaktive Stoff zumindest ein Magnesiumalkylethersulfat mit 1 bis 16 Ethylenoxideinheiten umfasst.

4. Zusammensetzung nach einem der vorhergehenden Ansprüche,
**dadurch gekennzeichnet, dass** sie mindestens 0,1 Gew.-% des ersten anionischen grenzflächenaktiven Stoffes, bezogen auf das Gesamtgewicht der Zusammensetzung, enthält.

5. Zusammensetzung nach dem vorherigen Anspruch, **dadurch gekennzeichnet, dass** sie 0,1 bis 10 Gew.-% des ersten anionischen grenzflächenaktiven Stoffes, vorzugsweise 0,2 bis 8 Gew.-% und noch bevorzugter 0,3 bis 2 Gew.-%, bezogen auf das Gesamtgewicht der Zusammensetzung, enthält.

6. Zusammensetzung nach einem der vorhergehenden Ansprüche, **dadurch gekennzeichnet, dass** der zweite anionische grenzflächenaktive Stoff unter den Salzen, besonders den Alkali- oder Erdalkalimetallsalzen, die von den Magnesiumsalzen verschieden sind, wie Natriumsalzen, Ammoniumsalzen, Aminsalzen, Aminoalkoholsalzen der folgenden Verbindungen ausgewählt ist: Alkylsulfate, Alkylethersulfate, Alkylamidoethersulfate, Alkylarylpolyethersulfate, Monoglyceridsulfate, Alkylsulfonate, Alkylamidsulfonate, Alkylarylsulfonate, α-Olefinsulfonate, Paraffinsulfonate, Alkylsulfosuccinate, Alkylethersulfosuccinate, Alkylamidsulfosuccinate, Alkylsulfoacetate, Acylsarcosinate und Acylglutamate und deren Gemische, wobei die Alkyl- und Acylgruppe dieser Verbindungen 6 bis 24 Kohlenstoffatome enthält und die Arylgruppe vorzugsweise Phenyl oder Benzyl bedeutet.

7. Zusammensetzung nach einem der vorhergehenden Ansprüche, **dadurch gekennzeichnet, dass** der zweite anionische grenzflächenaktive Stoff unter den Alkyl(C₆₋₂₄)monoestern von Polyglycosiddicarbonsäuren, wie Alkylglucosidcitraten, Alkylpolyglycosidtartraten und Alkylpolyglycosidsulfosuccinaten, Alkylsulfosuccinamaten, Acylisethionaten und N-Acyltauraten ausgewählt ist, wobei die Alkyl- oder Acylgruppe dieser Verbindungen vorzugsweise 12 bis 20 Kohlenstoffatome aufweist.

8. Zusammensetzung nach einem der vorhergehenden Ansprüche, **dadurch gekennzeichnet, dass** der zweite anionische grenzflächenaktive Stoff unter den Acyllactylaten, deren Acylgruppe 8 bis 20 Kohlenstoffatome enthält, Alkyl-D-galactosiduronsäuren und deren Salzen sowie den polyalkoxylierten Alkyl(C₆₋₂₄)ethercarbonsäuren, polyalkoxylierten Alkyl(C₆₋₂₄)aryl(C₆₋₂₄)ethercarbonsäuren, polyalkoxylierten Alkyl(C₆₋₂₄)amidoethercarbonsäuren und deren Salzen, insbesondere Verbindungen mit 2 bis 50 Ethylenoxideinheiten, und deren Gemischen ausgewählt ist.

9. Zusammensetzung nach Anspruch 6, **dadurch gekennzeichnet, dass** der zweite anionische grenzflächenaktive Stoff unter den Alkylsulfaten, Alkylethersulfaten und deren Gemischen insbesondere in der Form der Alkalimetallsalze, Erdalkalimetallsalze, Ammoniumsalze, Aminsalze und Aminoalkoholsalze ausgewählt ist.

10. Zusammensetzung nach einem der vorhergehenden Ansprüche, **dadurch gekennzeichnet, dass** sie mindestens 0,5 Gew.-% des (der) zweiten anionischen grenzflächenaktiven Stoffe(s), bezogen auf das Gesamtgewicht der Zusammensetzung, enthält.

11. Zusammensetzung nach Anspruch 10, **dadurch gekennzeichnet, dass** sie 0,5 bis 20 Gew.-% des (der) zweiten anionischen grenzflächenaktiven Stoffe(s), vorzugsweise 1 bis 20 Gew.-% und noch bevorzugter 3 bis 15 Gew.-%, bezogen auf das Gesamtgewicht der Zusammensetzung, enthält.

12. Zusammensetzung nach einem der vorhergehenden Ansprüche, **dadurch gekennzeichnet, dass** der amphotere oder zwitterionische grenzflächenaktiven Stoff ausgewählt ist unter:
- sekundären oder tertiären, aliphatischen Aminderivaten, bei denen die aliphatische Gruppe eine gerade oder verzweigte Kette mit 8 bis 22 Kohlenstoffatomen ist, die mindestens eine anionische Gruppe aufweist, wie beispielsweise Carboxylat, Sulfonat, Sulfat, Phosphat oder Phosphonat, und
- Alkyl(C₈₋₂₀)betainen, Sulfobetainen, Alkyl(C₈₋₂₀)amidoalkyl-(C₆₋₈)betainen oder Alkyl(C₈₋₂₀)amidoälkyl(C₆₋₈)sulfobetainen.

13. Zusammensetzung nach Anspruch 12, **dadurch gekennzeichnet, dass** der amphotere oder zwitterionische grenzflächenaktiven Stoff unter den Alkyl(C₈₋₂₀)betainen, Alkyl(C₈₋₂₀)amidoalkyl(C₆₋₈)-betainen und deren Gemischen ausgewählt ist.

14. Zusammensetzung nach einem der vorhergehenden Ansprüche, **dadurch gekennzeichnet, dass** sie mindestens 0,1 Gew.-% des (der) amphoteren oder zwitterionischen grenzflächenaktiven Stoffe(s), bezogen auf das Gesamtgewicht der Zusammensetzung, enthält.

15. Zusammensetzung nach Anspruch 14, **dadurch gekennzeichnet, dass** sie 0,1 bis 10 Gew.-% des (der) amphoteren oder zwitterionischen grenzflächenaktiven Stoffe(s), vorzugsweise 0,5 bis 8 Gew.-% und noch bevorzugter 1 bis 8 Gew.-%, bezogen auf das Gesamtgewicht der Zusammensetzung, enthält.

16. Zusammensetzung nach einem der vorhergehenden Ansprüche, **dadurch gekennzeichnet, dass** der ethoxylierte Sorbitanester unter den ethoxylierten Derivaten von Mono- und Polyestern von C₈-₃₀-Fettsäuren und Sorbitan ausgewählt ist, die 1 bis 50 Ethylenoxideinheiten aufweisen.

17. Zusammensetzung nach dem vorhergehenden Anspruch, **dadurch gekennzeichnet, dass** der ethoxylierte Sorbitanester unter den ethoxylierten Derivaten von Mono- und Polyestern von C₁₂₋₂₄-Fettsäuren und Sorbitan ausgewählt ist, die 4 bis 20 Ethylenoxideinheiten aufweisen.

18. Zusammensetzung nach dem vorhergehenden Anspruch, **dadurch gekennzeichnet, dass** es sich bei der Fettsäure des ethoxylierten Sorbitanesters um eine gesättigte Fettsäure handelt.

19. Zusammensetzung nach dem vorhergehenden Anspruch, **dadurch gekennzeichnet, dass** der ethoxylierte Sorbitanester unter ethoxyliertem Sorbitanmonolaurat mit 4 EO, ethoxyliertem Sorbitanmonolaurat mit 20 EO und deren Gemischen ausgewählt ist.

20. Zusammensetzung nach einem der vorhergehenden Ansprüche, **dadurch gekennzeichnet, dass** sie mindestens 0,5 Gew.-% ethoxylierten Sorbitanester, bezogen auf das Gesamtgewicht der Zusammensetzung, enthält.

21. Zusammensetzung nach dem vorhergehenden Anspruch, **dadurch gekennzeichnet, dass** sie 0,5 bis 10 Gew.-% ethoxylierten Sorbitanester, und vorzugsweise 2 bis 9 Gew.-%, bezogen auf das Gesamtgewicht der Zusammensetzung, enthält.

22. Zusammensetzung nach einem der vorhergehenden Ansprüche, **dadurch gekennzeichnet, dass** das kationische Polymer unter den Polymeren ausgewählt ist, die entweder als Teil der Polymerhauptkette oder an einem direkt daran gebundenen seitlichen Substituenten Einheiten enthalten, die primäre, sekundäre, tertiäre und/oder quartäre Aminogruppen aufweisen.

23. Zusammensetzung nach dem vorhergehenden Anspruch, **dadurch gekennzeichnet, dass** das kationische Polymer unter den Celluloseetherderivaten, die quartäre Ammoniumgruppen aufweisen, kationischen Cyclopolymeren, insbesondere Homopolymeren und Copolymeren von Dimethyldiallylammoniumchlorid, mit einem 2,3-Epoxypropyltrimethylammoniumsalz modifizierten Guargummen, quartären Polymeren von Vinylpyrrolidon und Vinylimidazol ausgewählt ist.

24. Zusammensetzung nach einem der vorhergehenden Ansprüche, **dadurch gekennzeichnet, dass** sie mindestens 0,01 Gew.-% kationische(s) Polymer(e), bezogen auf das Gesamtgewicht der Zusammensetzung, enthält.

25. Zusammensetzung nach dem vorhergehenden Anspruch, **dadurch gekennzeichnet, dass** sie 0,05 bis 10 Gew.-% kationische(s) Polymer(e) und vorzugsweise 0,1 bis 5 Gew.-%, bezogen auf das Gesamtgewicht der Zusammensetzung, enthält.

26. Zusammensetzung nach einem der vorhergehenden Ansprüche, **dadurch gekennzeichnet, dass** das amphotere oder zwitterionische Polymer unter den Polymeren ausgewählt ist, die statistisch in der Polymerkette verteilte Einheiten K und M enthalten, wobei K eine Einheit bezeichnet, die von einem Monomer mit mindestens einem basischen Stickstoffatom abgeleitet ist, und M eine Einheit bezeichnet, die von einem Säuremonomer mit einer oder mehreren Carbonsäuregruppen oder Sulfonsäuregruppen stammt, oder wobei K und M Gruppen bedeuten können, die von zwitterionischem Carboxybetain- oder Sulfobetainmonomeren abgeleitet sind, oder wobei K und M eine kationische Polymerkette bedeuten können, die primäre, sekundäre, tertiäre oder quartäre Aminogruppen enthält, bei der mindestens eine Aminogruppe eine Carbonsäuregruppe oder eine Sulfonsäuregruppe trägt, die über eine Kohlenwasserstoffgruppe gebunden ist, oder wobei K und M Teil einer Kette eines Polymers mit α,β-Dicarboxyethyleneinheit sind, bei der eine Carbonsäuregruppe mit einem Polyamin umgesetzt wurde, das eine oder mehrere primäre oder sekundäre Aminogruppen trägt.

27. Zusammensetzung nach dem vorhergehenden Anspruch, **dadurch gekennzeichnet, dass** das amphotere oder zwitterionische Polymer ein Polymer ist, das bei der Copolymerisation eines Monomers, das von einer Vinylverbindung mit Carbonsäuregruppe abgeleitet ist, wie Acrylsäure, Methacrylsäure, Maleinsäure, alpha-Chloracrylsäure, und eines basischen Monomers gebildet wird, das von einer substituierten Vinylverbindung abgeleitet ist, das mindestens ein basisches Atom enthält.

28. Zusammensetzung nach einem der vorhergehenden Ansprüche, **dadurch gekennzeichnet, dass** sie mindestens 0,01 Gew.-% amphotere oder zwitterionische Polymere, bezogen auf das Gesamtgewicht der Zusammensetzung, enthält.

29. Zusammensetzung nach dem vorhergehenden Anspruch, **dadurch gekennzeichnet, dass** sie mindestens 0,05 bis 10 Gew.-% amphotere(s) oder zwitterionische(s) Polymer(e) und noch bevorzugter 0,1 bis 5 Gew.-%, bezogen auf das Gesamtgewicht der Zusammensetzung, enthält.

30. Zusammensetzung nach einem der vorhergehenden Ansprüche, **dadurch gekennzeichnet, dass** sie mindestens zwei unterschiedliche Polymere enthält, die unter den kationischen Polymeren, den amphoteren Polymeren und den zwitterionischen Polymeren ausgewählt ist.

31. Zusammensetzung nach einem der vorhergehenden Ansprüche, **dadurch gekennzeichnet, dass** sie mindestens ein kationisches Polymer und mindestens ein amphoteres oder zwitterionisches Polymer enthält.

32. Zusammensetzung nach einem der vorhergehenden Ansprüche, **dadurch gekennzeichnet, dass** sie ferner mindestens ein Antischuppenmittel, wie beispielsweise Piroctone Olamine, Zink-Pyrithion, Salicylsäure, Selendisulfid und deren Gemische enthält.

33. Zusammensetzung nach dem vorhergehenden Anspruch, **dadurch gekennzeichnet, dass** sie 0,001 bis 10 Gew.-% des oder der Antischuppenmittel(s) und vorzugsweise 0,1 bis 5 Gew.-%, bezogen auf das Gesamtgewicht der Zusammensetzung, enthält.

34. Zusammensetzung nach einem der vorhergehenden Ansprüche, **dadurch gekennzeichnet, dass** sie ferner mindestens ein Silicon enthält.

35. Zusammensetzung nach einem der vorhergehenden Ansprüche, **dadurch gekennzeichnet, dass** das wässrige Medium aus Wasser oder einem Gemisch von Wasser und mindestens einem kosmetisch akzeptablen Lösemittel besteht.

36. Zusammensetzung nach Anspruch 35, **dadurch gekennzeichnet, dass** das Lösemittel unter den niederen Alkoholen mit 1 bis 4 Kohlenstoffatomen und den Polyolen ausgewählt ist.

37. Zusammensetzung nach einem der vorhergehenden Ansprüche, **dadurch gekennzeichnet, dass** sie ferner mindestens einen Zusatzstoff enthält, der unter den Wirkstoffen gegen Haarausfall, Oxidationsmitteln, Ceramiden und Pseudoceramiden, Vitaminen und Provitaminen und darunter Panthenol, pflanzlichen Ölen, tierischen Ölen, Mineralölen oder synthetischen Ölen, Wachsen, Sonnenschutzfiltern, anorganischen oder organischen Pigmenten, die farbig oder nicht farbig sind, Farbstoffen, Perlglanzstoffen und Trübungsmitteln, Maskierungsmitteln, Weichmachern, Lösungsvermittlern, Ansäuerungsmitteln, Alkalisierungsmitteln, anorganischen oder organischen Verdickungsmitteln, Antioxidantien, Hydroxysäuren, Parfums und Konservierungsmitteln ausgewählt ist.

38. Verwendung der Zusammensetzung nach einem der Ansprüche 1 bis 37 als Zusammensetzung für die kosmetische Behandlung oder Pflege der Haare oder der Haut.

39. Verwendung der Zusammensetzung nach einem der Ansprüche 1 bis 37 als Haarwaschmittel oder als Zusammensetzung, die vor oder nach einer Haarwäsche aufgebracht wird.

40. Verfahren zur kosmetischen Behandlung, **dadurch gekennzeichnet, dass** es das Auftragen einer wirksamen Menge einer Zusammensetzung nach einem der Ansprüche 1 bis 37 auf die Haare umfasst.
